# EUROPEAN PATENT APPLICATION

(11) **EP 0 893 123 A1**
(43) Date of publication of application: **27.01.1999**
(21) Application number: 96931314.7
(22) Date of filing: 10.09.1996
(51) Int. Cl.: A61K 33/00

(54) **AGENT FOR USE IN THE PREVENTION AND TREATMENT OF DISORDERS ASSOCIATED WITH VARIOUS TYPES OF AGEING**

(30) Priority: 13.09.1995 RU 95115500
(71) Applicant: Peganov, Eduard Maximovich, Moscow, 125171 (RU); Buldakova, Svetlana Leonidovna, St. Petersburg, 191128 (RU); Nikolaeva, Irina Sergeevna, Moscow, 103055 (RU); Shapovalova, Lyudmila Mikhailovna, Moskovskaya obl., 142450 (RU)
(72) Inventor: Peganov, Eduard Maximovich, Moscow, 125171 (RU); Buldakova, Svetlana Leonidovna, St. Petersburg, 191128 (RU); Nikolaeva, Irina Sergeevna, Moscow, 103055 (RU); Shapovalova, Lyudmila Mikhailovna, Moskovskaya obl., 142450 (RU)
(74) Representative: Popp, Eugen, Dr.
(86) International application number: RU9600255
(87) International publication number: WO9709991

(57) **Abstract**

The use of deuterium oxide as the means for preventive , maintenance and restorative therapy for conditions associated with ageing and/or pathogenic factors , manifested by dystrophic and degenerative changes in organs and tissues

## Description

### Area of engineering

The present invention is related to medicine, namely to an agent for use in the prevention and treatment of diseases, associated with ageing ,and also disorders, connected to accclerated decline of functions of organs and systems of organism under the influence of acute or chronic pathogenic factors.

With reference to a brain ageing is characterised:
on the behavioral level - by the impairment of memory, first of all on current events, and progressing dementia; on morphological - by the death of a significant part of nervous cells in different brain areas and degenerative changes of neurons and dendrites (Coleman P.D., Flood D.G., " Neuron numbers and dendritic extent in normal ageing and Alzheimer's disease ", Neurobiology of ageing, 1987, v.8, pp. 521-545); on physiological - by deficits of various systems of a brain (Greenamyre JB.. et al., " Alterations in L-glutamat binding in Alzheimer's and Huntington's diseases ", Science, 1985, v. 227, pp.1496-1499).

### Previous level of engineering

Preparation tacrin is now in use for the treatment of senile dementias, including that of Alzheimer type (Summers W.K . et. al., J. New Engl. J. Med., 1986, v.315, 20, p.1241).
Preparation amiridin (US, A 4735953, 1988, 514/313; DE, C, 3231571, 1983, A 61K 31/47) is also known.
The above-stated prepartions have rather high toxicity and side effects of cholinergic origin (tremor, disharrea, salivation). Besides toxic action of tacrin on liver may be expected at long application. Moreover, the treatment with these preparations has replacement, symptomatic character, not opposing to progressing development of disease per se.

### Disclosure of the invention

The invention was aimed at finding the means for preventive, maintenance and restorative treatment of disorders, associated with ageing, free from toxic and side effects.
This goal was achieved by the use of deuterium oxide 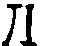 ₂O (heavy water) for preventive and restorative therapy of disorders, developing during ageing and/or under the action of pathogenic factors and connected with dystrophic and degenerative changes of organs and tissues. Deuterium oxide 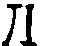 ₂O - natural stable isotope of usual water (H₂O), which prevents or slows down further deterioration of cells and restores their ability to carry out their specialised functions.
In the offered invention therapeutical effect is reached by the replacement of a part of body usual water by heavy water.
It is known, that the heavy water, practically not differing by its chemical properties from usual water, nevertheless renders isotopic effect on biological macromolecules, consisting in stabilisation of their native structure (Lobishev V.I., Kalinichenko L.P., " Isotopic effects of 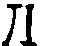 ₂O in biological systems, "Nauka "., Moscow, 1978), manifested by the increase of the temperature of proteins denaturation and accordingly by increase of their average life time (Hermans I.I., Scheraga H.A., " The thermally induced configurational change of ribonuclease in H₂O and 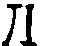 ₂O , Biochem. et biocphys. Acta, 1959, v. 36, 2, pp.534-535).
The cause of destructive and degenerative changes of organs and tissues can be , besides ageing, intensive and long lasting action of pathogenic factors, inducing accelerated loss of proteins (bacterial and virus infections, increased body temperature, intoxication, irradiation) It means, that the offered approach can be applicable not only in case of functions decline, associated with natural ageing, but also in case of accelerated pathological ageing, and also for treatment of patients, that have suffered intensive action of pathogenic factors and/or undergo their influence.
The use of deuterium oxide 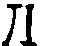 ₂O, unknown earlier from the level of engineering , as the means for the treatment of disorders of ageing and other pathologies, involving the loss of proteins by cells with subsequent dystrophic and degenerative changes of tissues, allows to slow down ageing process and gives an opportunity to withstand various infections.

### The brief description of figures of the drawings

Further the invention is illustrated by figures of the drawings, on which are represented:
- On figure 1: - electrophysiological characteristics of hippocampus of old rats before and after the treatment with heavy water;
- On figures 2,3: - amplitude of evoked potentials in mV before and after high frequency stimulation of hippocampus -tetanization - in rats of control group;
- On a figure 4: - amplitude of evoked potentials in mV before and after high frequency stimulation of hippocampus -tetanization - in rats of experimental group;
- On a figure 5: - influence of heavy water on the survival of the animals after the infection with pneumococcus;
- On a figure 6: - influence of heavy water on of animals after infection with pneumococcus and subsequent supporting therapy;
- On a figure 7: - influence of heavy water on the animals survival after injection of staphylococcus toxin.
- On a figure 8: - influence of heavy water on the septic process (pseudomonas aeruginosa).

### Variants of realisation of the invention

The declared means was studied in the experiments on animals. As an animal model of disorders of ageing we chose the decline of the phenomenon of long term potentiation of evoked responses in hippocampus of old rats.
As a model of the influence of pathogenic factors we chose generalised bacterial infection in adult mice and rats in its acute (bacterial shock) and chronic (septic process) forms and also toxic shock.

The following examples illustrate the invention:
Example 1. Long term potentiation (LTP, model of ageing)
The adequacy of the choice of the model is due the following factors:
- LTP phenomenon relates to function of memory, being an initial stage in a chain of processes, resulting in formation of traces of long-term memory, since the impairment of LTP is accompanied on the behavioral level by impairment of memory, first of all on current events (Landfield . W., Lynch G., " Impaired monosynaptic potentiation of in vitro hippocmpal slices aged memory-deficient rats., J. Gerontology, 1977, v.32, 5, pp. 523-533; Landfield P.W., Pilter T.A. Applegate M. D., " The aged hippocampus: A model system for studies on mechanisms of behavioral plasticity and brain ageing) The Hippocampus, 1986, v.3, pp. 323-367).
- the decrease of the content of various protein structures in old animals is reported (Monaghan D.T.et al is observed., " Age dependent loss of NMDA receptors in rodent brains ", Soc. Neurosci. Abstr.,1988, v.14, p.486; Angeluoci L., Imperato A.. " Age-dependent loss of muscarinic receptors and cholinergic impairment in the rats brain ", Neuroscience Letters, Suppl. 39, 1990, p. 8),
- the degree of dementia was reported to correlate with decrease of the membrane surface density of N-methyl-D-aspartate receptors, responsible for LTP, in patients with SDAT (Represa A., et al., " Is senile dementia of the Alzheimer type associated with hippocampal plasticity", Brain research, 1988, v.457, 355-359).
   the decrease of the rate of overall protein synthesis and, as a consequence, deficits of various proteins and products of non-protein synthesis is characteristic not only for a brain, but also for all organs and systems and are the mostly ascertained facts, concerning ageing, and recognised markers of ageing (Markides S.C. " Protein synthesis and degradation during aging and senescence ", Biol. Rev., 19836 v.58, 343-422; Rattan S. I., " Ageing and discase: proteins as the molecular link ", Perspect. Biol and Med., 1991, v.34, 4. Pp 526-533).

### Methods of research.

The experiments were performed on 20 old (29-30 months) outbred female rats. During 21 days control group (9 rats) received usual water as free drinking, and experimental group (11 rats) drank water, containing 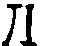 ₂O. Control weighing and visual observation of their behaviour was carried out. Then for 6 days both groups received usual water "to wash out " heavy water from the body in order to put both groups in equal conditions in the subsequent experiments. After that the experiment was carried out as follows: each day one control or one experimental animal was narcotised by urethane (1,3 g/kg) and decapitated. The hippocampal slices then were prepared, which were transferred to the special thermostatic chamber, where they were incubated during the experiment.
The chamber was perfused at the rate of 2 ml/minute with physiological solution (content in mM: NaCl-124; KCl-5,0; CaCl₂-2,5; MgSO₄- 2,4; KH₂PO₄-1,25; NaHCO₃-26; glucose-15; pH―7,4-7,5;), saturated with carbogen (95 % O₂ and 5 % CO₂). The slices were stored for 20-30 minutes at room temperature, then temperature was increased up to 28-29°C. Electrical activity was recorded in 1-1,5 hour after preparation of slices. The recording glass microelectrodes, filled with 2M solution of sodium citrate (electrical resistance 5-25 Mo 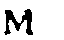 ) were inserted into the layer of pyramidal neurones. Electrical stimulation of Shaffer's collaterals was made through double-barrel glass microelectrodes, filled with physiological solution, with the resistance up to 2 Mo 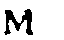 , by the rectangular pulses (amplitude up to 40 V, duration 0.2 msec, frequency 0,2-0,3 Hz). Data processing was made on the computer (" Electronics D3-28 ").

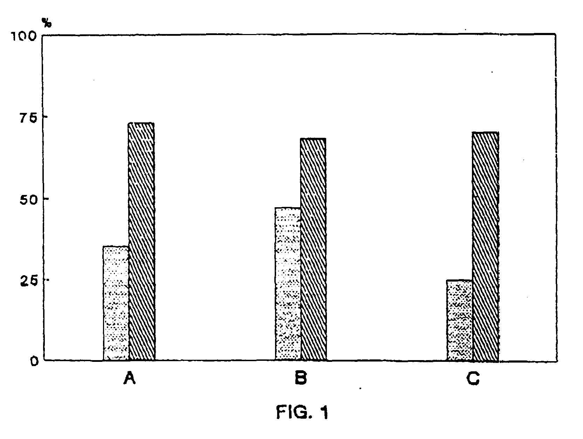

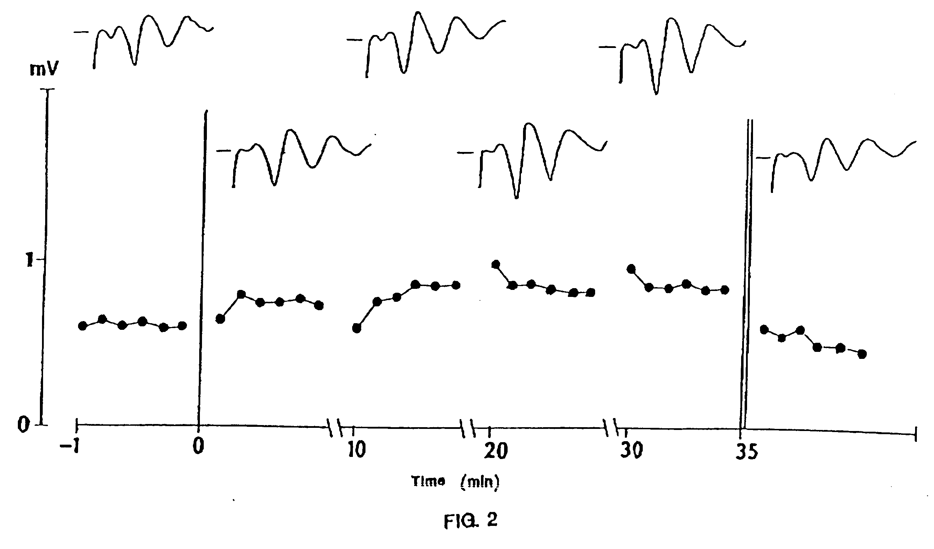

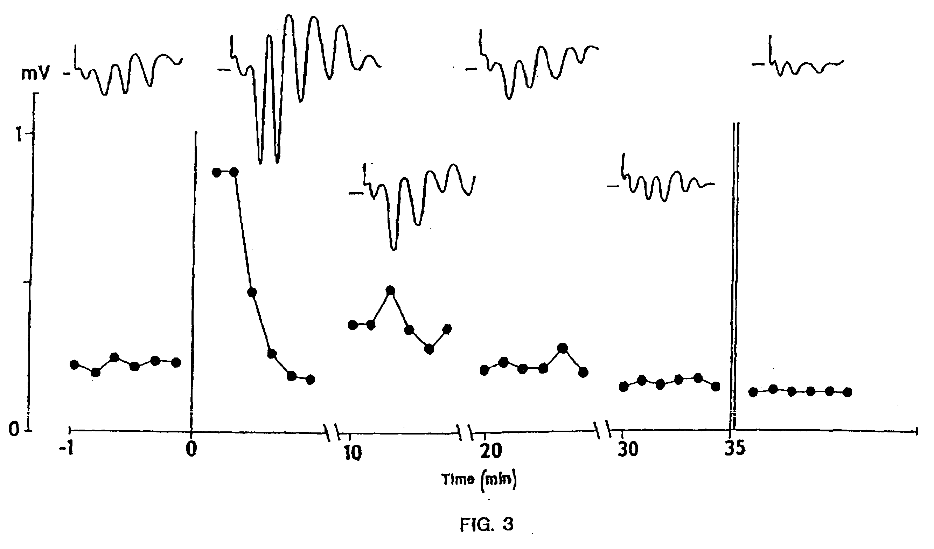

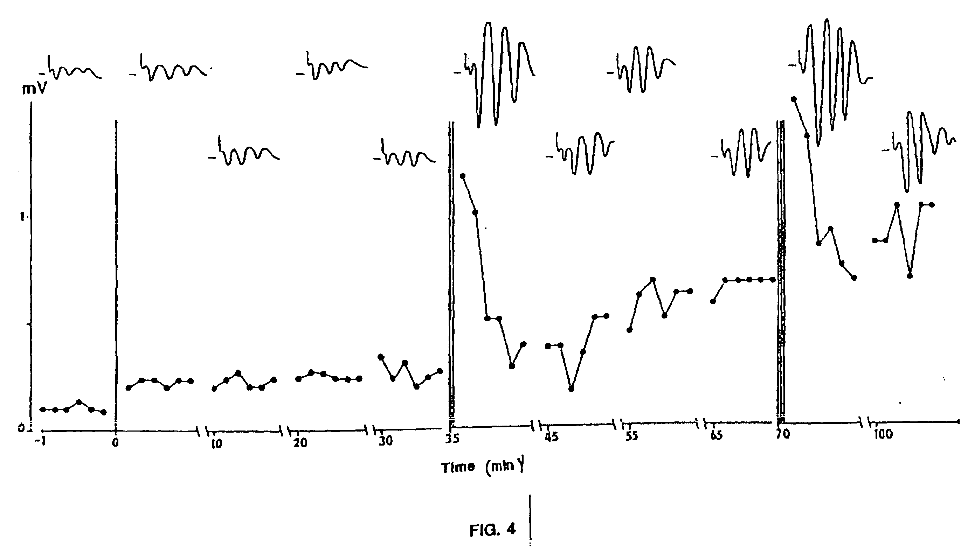

Results . On the average in control group weight of the animals was : at the 1-st day 365 ± 37 g, at 21-th day 380 ± 38 g. In experimental group: at the first day 373± 25 g, at 21-th day 375 ± 34 g. No statistically significant differences in animals weight and in the weight increment between the two groups was found out.
The study of electrophysiological characteristics was performed on 18 hippocampal slices (HS) in the experimental group (fig.1 , image by strokes) and on 14 HSs in the control group (fig.1, image by points) . The spontaneous pulse activity of pyramidal neurons of field CA1 was detected in 13 (72 %) of experimental HSs and in 5 (35 %) of control HSs(fig. 1, A). Focal evoked potentials (FEP) with preserved pre- and postsynaptic components were registered in 12 (67 %) experimental and in 7 (50 %) control HSs (fig.1, B). It should be noted, that FEP in slices of both groups contained, as a rule, 2-4 population spikes (PS) - components of focal potential, reflecting synchronous discharge of pyramidal neurones. PS in the experimental HSs on the average had amplitude 217 ± 55 mV, in control HSs 220 ± 54 mV. Frequency potentiation (FP) and short-term post-tetanic changes were observed in slices of both control and experimental groups.
At stimulation by frequency of 1 Hz the average increase of amplitude PS in control group was by 60 ± 27 %, in experimental group by 55±31 %. Essential differences between the control and experimental groups were found out in the study of a phenomenon long term post-tetanic potentiation (LTP). If the amplitude of PS after tetanization increased more, than by 25 %, and sustained during more than 30 minutes, LTP was considered to occur.
In control group LTP occured in 3 from 14 slices obtained from the brain of 2 of 8 (25 %) rats (Figure 1, C).In one of the animals LTP was expressed in the increase of PS amplitude by 45 % (Figure 2, on this figure and on figures 3 and 4 filled circles specify amplitude of evoked potential in mV, the vertical lined mark the moment of high-frequency stimulation - tetanization, the curves at the top part demonstrate original records of the evoked responses, made at corresponding moments of time). In the other animal LTP was expressed as the increase of the probability of the discharge of pyramidal neurone by 10 time (first slice) or as the appearance of the regular evoked response of pyramidal neurone after tetanization (second slice). In all the other slices tetanization induced either the depression of PS or no changes of PS amplitude (Figure 3). Repeated tetanization produced the depression of PS in all slices of control group (figure 3).

In experimental group LTP occurred in 10 of 18 HSs obtained from brain of 7 of 10 (70 %) rats (figure 1, C). On the average PS amplitude increased by 151 % + 26 %. Repeated tetanization in all cases resulted in further increase of the potentiated response. On a figure 4 an example of further growth of the potentiated response is shown: after second tetanization the increment was 730 % and after the third -1200 %.

### Example 2. Bacterial shock model

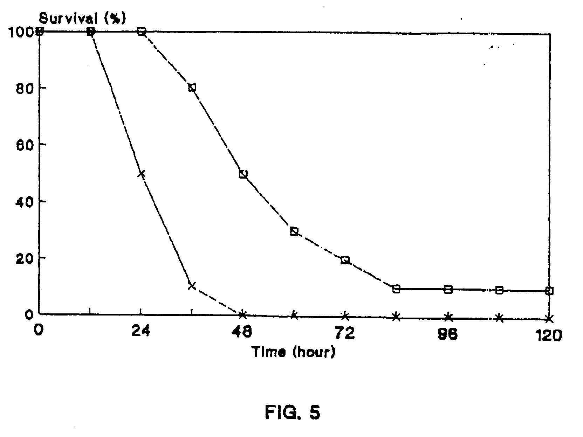

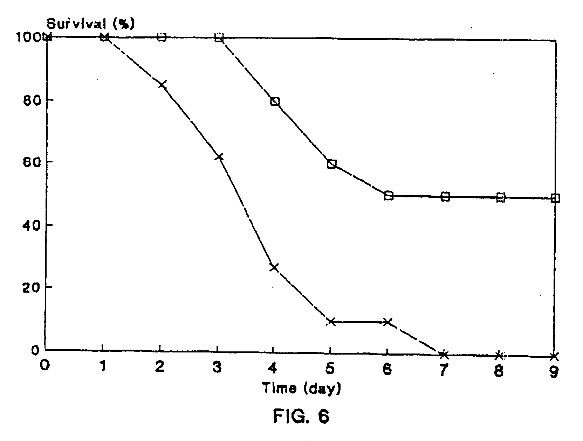

Bacterial shock in animals (outbred white mice) was induced by single intraperitoneal injection of daily culture of various bacteria in minimal lethal doze ( was defined before the experiment) . Experimental animals were allowed to drink diluted 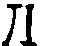 ₂O during 2-4 days before the experiment in order to set desired body content of heady water to the moment of culture injection. Fig 5. shows graphically presented results of the experiment with pneumococcus culture. As can be seen, the dying of experimental animals (10 mice, image ' -o-' ) was delayed by about 24 hours with respect to that of control animals group (10 mice, '-x-') and while 100% of control animals died in 48 hours 50% of experimental animal were still alive.
In the experiment shown in fig.6 animals of both groups (10 mice in each) were subjected to additional treatment during state of shock directed to maintenance of blood circulation, that allowed to slow down the rate of dying of control animals. One can see, that as in the experiment described above, the dying of animals treated with D₂O was delayed ( in this case by 48 hours) and half of them could overcome the shock and survived.

### Example 3. Toxic shock model.

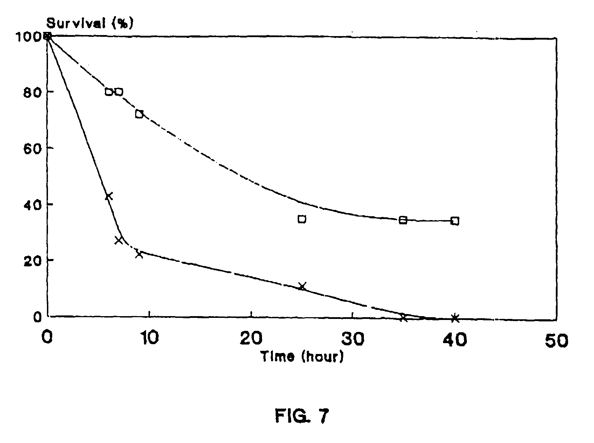

The scheme of the experiment was the same as in Example 2 . The experiments were performed on outbred white rats (control group 9 rats, experimental group 11 rats). The results of the experiment with injection of minimal lethal doze of staphylococcus toxin are presented on fig.7.
As as in the case of bacterial shock there was a delay of the time of dying and survival of 40% animals, treated with heavy water and in this case without additional measures of therapy.

### Example 4. Model of sepsis.

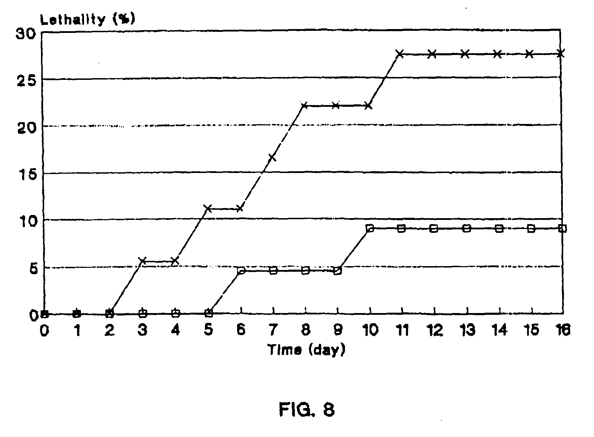

The effects of D₂O on septic process was studied on outbred white rats ( weight 180-200 g, 18 animals in control group, 22 animals in experimental group). Septic process in animals was induced according to previously developed technique by single intramascular injection of 0.3 ml of daily culture of standard pseudomonas aeruginosa strain N 450 in 10% CaCl₂ solution. Bacterial content was 7x10⁹ bodies per 1 ml. Infusion of calcium chloride led to formation of necrotic zone at the site of injection that prevented incapsulation of infected site thus allowing the propagation of infection and formation of metastatic pyemic sites in various organs. The development of infection process was much slower than in the case of bacterial shock and it became similar to the development of septic disease in man. Characteristic signs of septic process -metastatic pyemic sites- could be seen on macro- and microscopic level in kidney, lungs, spleen and liver. Postmortem section of animals showed dystrophic changes in organs .
Lethality of animals served as a criterion of the influence of D₂O on infection process. As earlier, experimental animals consumed D₂O before (during 2 days) and after (during 9 days) infection. The use of heavy water resulted in the delay of lethal outcomes by 3 days and in significant (by 3 times) decrease of lethality (fig. 8).

Thus as a result of treatment with heavy water 70% of old rats showed LTP with relative increase of evoked responses after tetanization characteristic of adult healthy animals. However, qualitative side of the effect may be even more important than its qualitative side: restoration of the reaction on repeated tetanization observable only in young animals.

In accordance with time schedule of electrophysiological experiments the last three treated animals, which showed highly expressed restored LTP phenomenon, were taken for testing on 19-th, 20-th and 21-th day after starting of measurements (that is on 25-th, 26-th and 27-th days after D₂O cancellation). In means that restored phenomenon of LTP persisted long (about month) after cancellation of treatment. In other words, three weeks long consumption of D₂O allowed old rats not only to completely restore but also to maintain the function of all parts of LTP mechanism.

The presence of heavy water in the body of infected animals allowed to slow down dying of animals under the conditions of bacterial and toxic shock and helped animals to withstand infection in chronic conditions.

### Field of use

The invention may be used for restorative and preventive therapy of diseases of ageing , in particular of senile dementia, including that of Alzheimer type , and also for conditions of pathogenic influences for therapy of pathologies manifested by dystrophic and degenerative changes in organs and tissues.

## Claims

1. Pharmaceutical mixtures, containing deuterium oxide, for use as a preventive, maintenance and restorative therapy for conditions associated with ageing and/or pathogenic factors and manifested by dystrophic and degenerative changes in organs and tissues.

2. Pharmaceutical mixtures, according to Claim 1, characterized by that they are used in the form of liquid deuterium oxide, diluted by usual water, delivered orally, intravascularly , rectally or intracavitarily to replace some water in the body by deuterium oxide.

3. Pharmaceutical mixtures, according to Claim1, characterized by that they are used in form of solutions, emulsions, greases or creams for external application.

4. Pharmaceutical mixtures, according to Claims 1-3, characterized by that they contain additionally other drugs and anti tumor agents.
